# FASCICULE DE BREVET EUROPEEN

(11) **EP 1 449 577 B1**
(45) Date de publication et mention de la délivrance du brevet: **05.05.2010**
(21) Numéro de dépôt: 04075976.3
(22) Date de dépôt: 05.10.1994
(51) Int. Cl.: B01F 17/00, C07C 233/36, C07C 233/38, C11D 1/88, C07D 233/02

(54) **Amphoacétates tensio-actifs de plus grande pureté dérivés de l'imidazoline.**
Imidazolin abgeleitete Amphoaectate-Tenside von höherer Reinheit.
Imidazoline derived amphoacetates surfactants of higher purity.

(30) Priorité: 12.10.1993 US 135094
(43) Date de publication de la demande: 25.08.2004
(62) Demande divisionnaire de: 94402226.8
(73) Titulaire: RHODIA INC., Cranbury, New Jersey 08512 (US)
(72) Inventeur: Desai, Bharat, Bel Mead New Jersey 08502 (US); Lees, Peter, Birkenshaw Bradford (GB); Ricca, Jean-Marc, Orchard Bel-Air (SG); Tracy, David J., Plainsboro New Jersey 08536 (US)
(74) Mandataire: Boittiaux, Vincent

(56) Documents cités:
- EP-A- 0 269 940
- EP-A- 0 373 491
- WO-A-94/13621
- DE-A- 2 725 780
- DE-A- 3 639 752
- DE-A- 4 307 709
- GB-A- 930 296
- US-A- 4 269 730

## Description

La présente invention concerne des amphoacétates tensio-actifs de plus grande pureté, dérivés de l'imidazoline, caractérisés par une amélioration de la régularité de leur composition, de la douceur et de l'activité de surface et par une amélioration de leurs procédés de préparation.

Les amphoacétates tensio-actifs, dérivés de l'imidazoline sont utilisés sur une grande échelle comme base tensio-active dans les formulations cosmétiques tels que shampooings, produits de nettoyage détergents et autres en raison de leur douceur, de la sécurité qu'ils offrent et de l'absence de tout effet irritant sur la peau et les yeux.

Les amphoacétates tensio-actifs, dérivés des imidazolines, constituent une famille de tensio-actifs caractérisée par la présence de motifs transitoires à charges positives et négatives. Selon le pH du milieu, ils peuvent, par conséquent, agir comme tensio-actifs anioniques, cationiques ou non-ioniques.

Les amphoacétates tensio-actifs présentent d'excellentes propriétés tensio-actives telles que réduction de la tension de surface, faibles valeurs pc-20 (efficacité tensio-active / quantité de tensio-actif nécessaire pour abaisser l'activité de surface de 20 unités), excellent pouvoir moussant, mouillant et autres. Ils sont compatibles à la fois avec les tensio-actifs cationiques et les tensio-actifs anioniques. En raison de leur biodégradabilité, de l'absence d'irritation de la peau et de leur aptitude incomparable à réduire le pouvoir irritant de tensio-actifs plus agressifs, tels que les éther sulfates , les amphoacétates tensio-actifs sont utilisés de plus en plus comme tensio-actifs secondaires dans l'industrie de l'hygiène corporelle. Par ailleurs, en raison de leur résistance à l'hydrolyse, de leur compatibilité avec les électrolytes et de leur excellente hydrotropie, ils peuvent être employés aussi dans les formulations de produits de nettoyage à usages domestiques et industriels.

Il existe plusieurs types différents de tensio-actifs amphotères, dérivés de l'imidazoline, par exemple, les propionates, les sulfonates et les acétates. Ils sont obtenus en faisant réagir un dérivé approprié de l'imidazoline sur un agent approprié contenant le groupe fonctionnel voulu. Par exemple, les amphoacétates peuvent être obtenus de la façon suivante :
1) on forme une imidazoline substituée en faisant réagir une aminoéthyl alkanol amine ou une éthylène alkylène triamine sur un acide gras 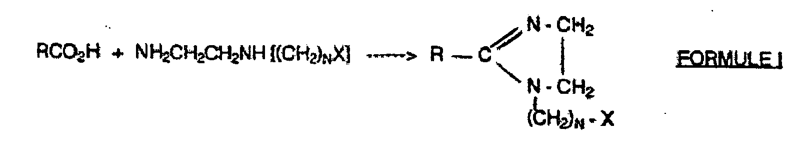
2) on hydrolyse l'imidazoline en une amido amine et l'on fait réagir ensuite (ou simultanément) lesdits composés sur un agent d'alkylation, tel que l'acide monohalogénoacétique ou son sel de sodium en présence d'hydroxyde de sodium.

On a pensé que les tensio-actifs amphotères, dérivés de l'imidazoline, peuvent contenir un mélange de différentes familles de tensio-actifs. Les structures suivantes ont été citées dans la littérature :

### AMPHOMONOACETATE

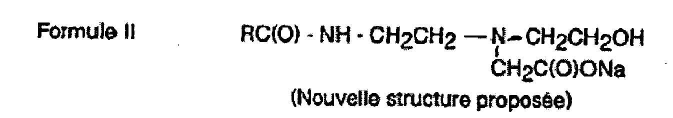

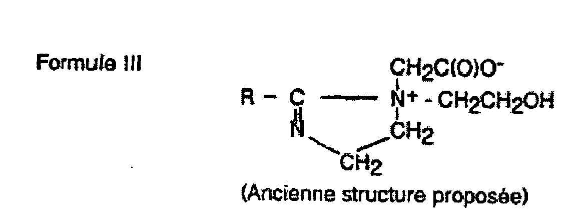

### AMPHODIACETATE

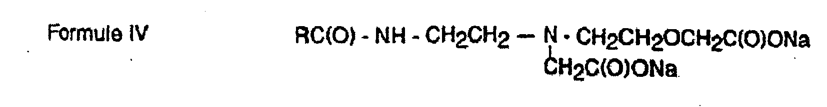

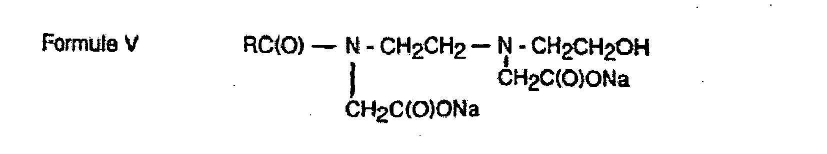

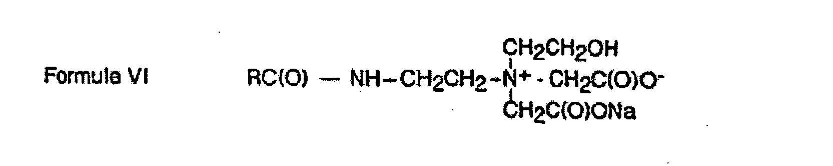

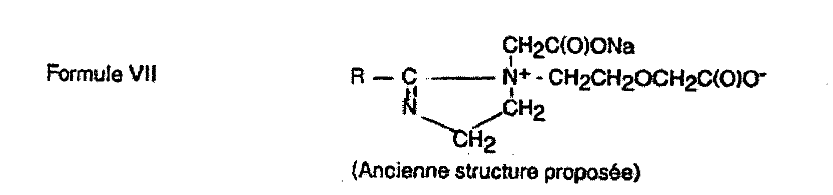

Dans ces formules, R représente des groupes coco ou d'autres groupes alkyle appropriés.

Le dictionnaire "Cosmetic Toiletry and Fragrance Association International Cosmetic Ingredients Dictionary" décrit les amphoacétates et les amphodiacétates comme étant deux familles distinctes de tensio-actifs amphotères dérivés de l'imidazoline. Ces derniers sont obtenus en faisant réagir une imidazoline dans des conditions d'alcoylation (carboxylation) avec soit un équivalent (ou léger excès) soit deux équivalents (ou un excès) d'un agent alcoylant approprié, par exemple, du monochloroacétate de sodium. Les alkyles correspondants peuvent être des chaînes coco, lauryle (coco ou lauryl amphoacétates ou diacétates) ou choisis parmi d'autres acides gras suivant l'application recherchée. De plus, étant donné que l'imidazoline de départ est asymétrique par nature, les deux types de dérivés peuvent être obtenus à partir de l'une ou de l'autre des amines possibles dérivant des imidazolines. C'est la raison pour laquelle les deux types de structures "amine primaire/amide tertiaire" et "amine secondaire/amide primaire" sont mentionnés dans la littérature.

L'agent d'alcoylation, le monochloroacétate de sodium, utilisé couramment pour préparer les amphoacétates et les diacétates, est un réactif chimique qui, dans des conditions de réaction, peut subir un certain nombre de différents types de réactions :
1. Réaction avec les fonctions amino de l'imidazoline ou des imidazolines hydrolysées pour donner des espèces mono ou polycarboxylées.
2. Réaction avec l'eau pour donner les dérivés correspondants de l'acide glycolique ou réaction avec l'acide glycolique pour obtenir des diglycolates.
3. Réaction avec les groupes hydroxyéthyles du dérivé de l'imidazoline pour obtenir les carboxyméthyl éthers correspondants.

Parmi les réactions possibles indiquées ci-dessus, seules "1" et "3" sont susceptibles de conduire à la formation des espèces amphotères recherchées alors que les réactions du type 2 donnent des sous-produits indésirables (glycolates/diglycolates), réduisant ainsi la quantité d'agent d'alcoylation disponible pour la réaction.

Récemment, après la mise au point d'une nouvelle méthode d'analyse, on a constaté que les coco/lauryl amphoacétates du commerce contiennent (en plus du chlorure de sodium) les principaux composants organiques suivants :
Amphoacétate (dérivé de l'amine secondaire/amide primaire, Formule II)
Amido amine non alcoylée correspondante
Glycolate/diglycolate
Monochloroacétate de sodium résiduel

Les résultats ci-dessus reposent sur l'utilisation de la spectroscopie par résonance magnétique nucléaire (¹H, 13C), de la chromatographie en phases liquide et gazeuse et de l'électrophorèse capillaire.

Le tableau suivant donne les résultats obtenus à la suite de l'analyse de trois amphoacétates du commerce :

| | rapport amphoacétate / amidoamine non alcoylée | acide glycolique % |
|---|---|---|
| Produit du commerce I | 75 / 25 | 2,6 |
| Produit du commerce II | 63 / 37 | 2,4 |
| Produit du commerce III | 83 / 17 | 2,0 |

Les résultats ci-dessus montrent que les produits du commerce contiennent tous une quantité significative de produit non alcoylé. La présence de produit non alcoylé exerce une action négative sur l'activité de surface.

L'excès de monochloroacétate de sodium conduit aussi à la formation d'un sous-produit, l'acide glycolique, sous la forme de glycolate de sodium. Ce sous-produit est indésirable, car l'acide glycolique ne contribue en rien aux propriétés tensio-actives du composé recherché.

Le brevet US n° 4 269 730 enseigne un procédé de préparation de tensio-actifs amphotères de plus grande pureté dans lequel on chauffe à une température comprise entre 70 et 80°C un mélange constitué d'imidazoline substituée essentiellement pure, de 1,0 à 2,5 équivalents molaires de sel de chloroacétate par mole d'imidazoline, et de l'eau, la teneur en matières solides étant comprise entre 20 et 50 %, jusqu'à ce que le chloroacétate soit consommé pour l'essentiel, on ajoute ensuite de 1,0 à 2,5 équivalents molaires de NaOH sur la base du sel de chloroacétate et on chauffe à une température allant de 70 à 80°C, au moins jusqu'à ce que tous les cycles imidazoline soient ouverts et forment une solution aqueuse limpide contenant de 15 à 35 % de matières solides. Contrairement aux enseignements de ce document, on a trouvé que le produit selon ce brevet contient toujours des quantités de sous-produits qui affectent l'activité de surface du produit final.

En contrôlant soigneusement les conditions de la réaction, on peut obtenir des amphoacétates tensio-actifs, dérivés de l'imidazoline, entièrement alcoylés pour l'essentiel, même si on met en oeuvre un excès relativement faible de monochloroacétate de sodium par rapport à l'imidazoline (1,0-1,5 : 1). Les produits obtenus présentent des activités tensio-actives qui sont comparables à celles que présentent actuellement les amphodiacétates disponibles dans le commerce, mais qui contiennent des taux beaucoup plus faibles de glycolate et de chlorure de sodium. Les produits de l'invention sont, par ailleurs, essentiellement exempts d'amido amines, si on les compare aux amphoacétates classiques, alors qu'ils contiennent des quantités comparables de chlorure de sodium et des quantités plus faibles de dérivés de l'acide glycolique. Ces amphoacétates de plus grande pureté présentent des propriétés tensio-actives supérieures à celles des produits conventionnels.

Selon l'invention, on met à disposition des amphoacétates tensio-actifs améliorés, dérivés de l'imidazoline, qui sont caractérisés par une plus grande pureté et présentent une meilleure activité de surface mise en évidence par une amélioration des pouvoirs moussant et mouillant, de la détergence et une réduction plus importante de la tension de surface. Ces amphoacétates tensio-actifs améliorés peuvent être obtenus par de nouveaux procédés qui comprennent la mise en oeuvre de pH contrôlés pendant toute la durée de la réaction et, notamment, pendant la phase d'alcoylation du procédé. Le pH peut être contrôlé en ajoutant lentement ou automatiquement une base appropriée, tel que de l'hydroxyde de sodium, en réponse à un système de mesure du pH, en vue de maintenir le pH constant. Cela peut être effectué également en procédant à toute une série d'additions étagées d'une base, par exemple, d'hydroxyde de sodium, calculées de façon à maintenir le pH à l'intérieur de la fourchette requise. Il a été trouvé aussi que les produits ayant une plus grande pureté peuvent être obtenus en soumettant l'imidazoline à des conditions favorisant l'ouverture des cycles avant l'alcoylation, suivies de la réaction avec l'agent d'alcoylation, par exemple, le monochloroacétate de sodium, dans des conditions soigneusement contrôlées. En conduisant la réaction de l'imidazoline ou de son dérivé à cycle ouvert avec le sel de l'acide halogénoacétique dans des conditions où le pH est contrôlé, on augmente notablement le rendement de la réaction d'alcoylation, ce qui aboutit à la formation de quantités moindres d'acide glycolique. L'acide glycolique se forme à partir du sel d'halogénoacétate et est généralement compensé par un excès d'acétate. Le procédé selon l'invention permet de mettre en oeuvre des rapports molaires plus faibles entre l'imidazoline substituée ou son dérivé et les sels d'halogénoacétate, soit inférieurs à 1 : 1,5 et voisins de 1 : 1, alors que l'on obtient toujours un produit qui, pour l'essentiel, est entièrement alcoylé.

Le contrôle soigneux du pH et de la température pendant la durée de la réaction permet à la réaction de se dérouler avec une quantité moindre de sel de monohalogénoacétate de sodium et d'obtenir ainsi un produit de plus grande pureté (moins de sous-produits amide non alcoylée, acide glycolique, NaCl et sel d'halogénoacétate résiduel). Les produits selon l'invention, outre qu'ils sont économiquement plus intéressants que les produits obtenus par purification des produits préparés selon le procédé de l'état de la technique, présentent des propriétés tensio-actives supérieures et une plus grande flexibilité dans les formulations.

Les imidazolines de départ, mises en oeuvre dans le procédé de l'invention peuvent être représentées par la formule suivante : 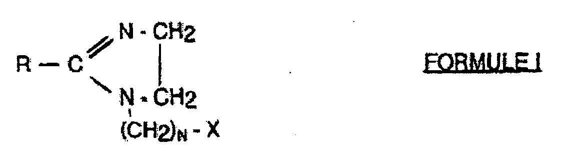 dans laquelle R est un radical aliphatique contenant de 5 à 19 atomes de carbone par molécule, X représente OH ou NH₂ et N est un nombre entier de 2 à 4. R est, de préférence un radical aliphatique contenant en majorité de 8 à 18 atomes de carbone environ par molécule, X représentant OH et N étant égal à 2.

En raison de la chimie en question, les composés selon la formule ci-dessus contiennent généralement des mélanges de différents radicaux R dans les fourchettes définies plus haut. Dans l'un des modes de réalisation préférentiels de l'invention, R est un mélange de radicaux aliphatiques saturés et insaturés, dérivés de l'huile de coco ou de sources d'huiles naturelles similaires, telle que l'huile de palme, ou de sources de graisses animales, tel que le suif. Dans ce cas, chaque R est un mélange de radicaux alkyles contenant environ de 5 à 18 atomes de carbone. Dans un produit plus préférentiel, le mélange de radicaux alkyle dérive d'une fraction saturée d'huile de coco ou d'huile végétale naturelle similaire. Dans le cas de l'acide gras de l'huile de coco, chaque radical R varie entre environ 6 et environ 18 atomes de carbone. Les fourchettes indiquées couvrent environ 90 % des groupes R du composé. Etant donné que lesdits groupes R dérivent de sources naturelles, ils peuvent contenir de faibles quantités d'autres chaînes carbonées. Par ailleurs, on peut mettre en oeuvre des imidazolines à base d'acides carboxyliques purs, tels que, par exemple, l'acide laurique, ou d'autres coupes, en fonction de l'application particulière.

Les imidazolines mises en oeuvre selon la présente invention devraient se présenter sous une forme essentiellement pure. "Essentiellement pure", signifie exempt, dans une large mesure, d'acides gras, d'aminoéthyléthanol amine, d'amido esters et de diamides. Pour les besoins de l'invention, on peut accepter la présence d'amido amines. Tout procédé approprié de préparation des imidazolines peut être mis en oeuvre.

Comme exemples d'imidazolines de départ, on peut citer la 2-heptylimidazoline, la 2-dodécylimidazoline, la 2-heptadécylimidazoline, la 1-hydroxyéthyl-2-dodécylimidazoline, la 1-hydroxyéthyl-2-heptadécylimidazoline et autres similaires. Comme exemples d'acides gras purs ou de mélanges d'acides gras que l'on peut mettre en oeuvre pour préparer les imidazolines, on peut citer l'acide gras de l'huile de coco, l'acide de l'huile de palme, les acides gras caprique, caproïque, caprylique, hexadécadiénoïque, laurique, linoléique, linolénique, margarique, myristique, myristoléique, oléique, palmitique, palmitoléique, stéarique et autres similaires.

En ce qui concerne la mise en oeuvre pratique du procédé de l'invention, on émet l'hypothèse selon laquelle, l'imidazoline réagit dans des conditions qui favorisent l'ouverture du cycle de l'imidazoline avant que n'intervienne la réaction d'alcoylation. Dans les phases initiales, la réaction est généralement conduite de façon à favoriser l'ouverture du cycle. Dans l'une des réalisations de l'invention, l'imidazoline est chauffée à un pH élevé compris entre environ 8,5 et environ 10 pour faciliter l'ouverture d'au moins une majorité de cycles de l'imidazoline. Dans une autre réalisation, l'imidazoline peut être additionnée de monohalogénoacétate à un pH élevé dans des conditions favorisant l'ouverture du cycle. Dans une troisième réalisation, le monohalogénoacétate est ajouté à l'imidazoline pendant l'addition de base dans des conditions telles que le pH est maintenu dans la fourchette comprise entre environ 9 et environ 10 pendant la durée de l'addition. Cette théorie est présentée dans le but d'essayer d'expliquer l'invention, mais la demanderesse ne souhaite pas en être prisonnière.

Dans la mise en oeuvre pratique de l'invention, on chauffe l'imidazoline avec un sel de monohalogénoacétate. L'halogénoacétate se présente de préférence sous la forme d'une solution aqueuse avant d'être ajouté à l'imidazoline. Pour ce faire, il est commode de préparer le sel à partir de l'acide juste avant la réaction. L'avantage que présente cette façon de procéder réside dans le fait que le sel peut être préparé avec un excès de base permettant de neutraliser l'acide hydrohalogéné qui se forme pendant la réaction de imidazoline avec le sel d'halogénoacétate. Le pH en excès varie entre environ 8 et environ 10. Il va de soi que le sel d'halogénoacétate peut être acheté et préparé ailleurs, dissous dans de l'eau et mis en oeuvre tel quel ou, de préférence, avec la quantité de base ajoutée correspondant à l'excès dont il a été question plus haut.

Comme exemples de sels de monohalogénoacétate appropriés dans lesquels la fraction cationique est un ion métal alcalin, on peut citer le monochloroacétate de sodium, le monobromoacétate de sodium, le monochloroacétate de potassium et le monobromoacétate de potassium. Les monohalogénoacétates préférentiels sont les sels de sodium et de potassium de l'acide monochloroacétique.

Comme exemples d'alcalis appropriés pouvant être mis en oeuvre dans le procédé de l'invention, on peut citer l'hydroxyde de sodium, l'hydroxyde de potassium, le carbonate de sodium, le carbonate de potassium, le bicarbonate de sodium, le bicarbonate de potassium et autres similaires. L'alcali est de préférence l'hydroxyde de sodium et/ou de potassium.

Le rapport molaire de l'acide monohalogénoacétique ou de sa forme sel à l'imidazoline ou à l'amine est de préférence supérieur à 1. Lorsque les quantités sont inférieures à 1, l'acide monohalogénoacétique, présent en quantité insuffisante, assure une alcoylation complète, le produit étant alors contaminé par l'amido amine qui ne possède aucune activité de surface. Si l'on met en oeuvre un rapport trop élevé, le produit contiendra un excès d'acide glycolique puisque le sel de monohalogénoacétate réagira avec la base pour la transformer en acide glycolique. L'un des résultats surprenants de la présente invention réside dans le fait que le rapport peut être maintenu aussi bas que possible avec un léger excès permettant de conduire la réaction à son terme tout en obtenant une alcoylation quasiment complète. Il est possible grâce à la présente invention de maintenir le rapport aussi bas que 1,05 : 1. Le rapport varie de préférence entre environ 1,05 : 1 et environ 1,5 : 1, plus préférentiellement entre 1,05 : 1 et environ 1,4 : 1 et, plus préférentiellement encore entre 1,05 et environ 1,2 : 1.

La réaction est conduite en règle générale à une température qui aboutit à la réaction comme il est bien connu dans l'industrie. Les températures de la réaction principale peuvent aller jusqu'à 95 °C ; elles sont comprises, de préférence, entre environ 50°C et environ 95°C. Dans l'un des modes de réalisation de l'invention, on a mis en oeuvre une température de l'ordre de 95°C. Dans un autre mode de réalisation, on a constaté que les températures comprises entre environ 70°C et environ 80°C étaient efficaces et, dans un troisième, on a mis en oeuvre une température de l'ordre de 50°C. D'une manière plus préférentielle, la température est comprise entre environ 75°C et environ 85°C. La réaction peut être chauffée dès que l'on considère que la réaction principale est terminée afin de s'assurer que la réaction est bien complète. Pendant cette phase de la réaction, les températures peuvent monter jusqu'à 100°C.

Les temps de réaction sont suffisants pour permettre la réalisation de chacune des phases désirées de la réaction et peuvent être déterminés facilement par l'homme du métier.

En règle générale, on mélange l'acide monohalogénoacétique ou son sel et l'imidazoline d'une manière aussi rapide et pratique que possible pour que ledit mélange des réactifs soit complet. Le contrôle du pH étant essentiel, les réactifs et, notamment, la base, sont ajoutés à une vitesse permettant de prévenir toute augmentation du pH au-dessus d'environ 10. On prend toutes les précautions nécessaires pour éviter des "points chauds" (hot spots) en certains endroits au cours de l'addition de la base. On procède à une addition de la base par incréments afin d'éviter toute augmentation brutale du pH.

Le contrôle soigneux du pH et de la température pendant la réaction permet d'effectuer la réaction avec une quantité moindre de sel de monohalogénoacétate de sodium et d'obtenir un produit de plus grande pureté (moins de sous-produits amide non alcoylée, acide glycolique, NaCl et sel d'halogénoacétate résiduel). Les compositions selon la présente invention sont caractérisées par des teneurs en amide non alcoylée inférieures à environ 3,5 %, de préférence inférieures à environ 2,0 % et plus préférentiellement encore inférieures à environ 0,5 % par rapport aux matières actives. Les compositions selon la présente invention sont caractérisées aussi par des teneurs en acide glycolique inférieures à environ 4,5 %, de préférence inférieures à environ 3,5 % et plus préférentiellement encore inférieures à 2,5 % d'acide glycolique par rapport aux matières actives. Les compositions selon la présente invention sont encore caractérisées par des teneurs en sel de métaux alcalins, tel que, par exemple, en chlorure de sodium, inférieures à environ 27 %, de préférence inférieures à environ 23 % et plus préférentiellement encore inférieures à 20 % par rapport aux matières actives. En raison de l'amélioration de leur pureté, les produits selon l'invention, outre qu'ils sont économiquement plus intéressants que les produits obtenus par purification des produits préparés selon le procédé de l'état de la technique, présentent des propriétés tensio-actives supérieures et une plus grande flexibilité dans les formulations.

Les amphoacétates tensio-actifs selon la présente invention sont extrêmement doux et n'irritent ni les yeux ni la peau. Ils présentent aussi une une augmentation de la vitesse mouillante, une diminution plus importante de la tension de surface, des propriétés moussantes et de stabilisation de la mousse élevées, une faible toxicité et une excellente compatibilité avec les autres tensio-actifs anioniques, ioniques et non ioniques. Ces produits sont stables dans une large fourchette de pH et sont biodégradables. Ces propriétés font qu'ils peuvent être mis en oeuvre dans des produits allant des cosmétiques aux applications industrielles ; ils peuvent être utilisés partout où les amphoacétates tensio-actifs de ce type ont trouvé une application. Ces produits conviennent tout particulièrement pour les shampooings, notamment les shampooings pour bébés, les shampooings corporels, y compris les bains à bulles, les savons, les gels de bain, les gels et lotions de mise en forme de la chevelure, les crèmes et lotions pour la peau, les crèmes et lotions de démaquillage, les détergents, les détergents pour la vaisselle et autres produits de lavage et cosmétiques en contact avec la peau.

Les exemples suivants vont permettre d'illustrer plus complètement la présente invention. Les pourcentages de pureté des produits qui sont préparés dans les exemples ainsi que les pourcentages qui sont indiqués dans les revendications, par rapport aux matières actives, sont exprimés en poids par rapport à la quantité de produit actif présent dans le produit, déterminée en soustrayant des matières solides telles quelles à l'issue de la réaction la quantité de chlorure de sodium, d'acide glycolique et d'amido amine.

### EXEMPLE 1

Cet exemple illustre le procédé de la présente invention mettant en oeuvre différentes phases d'addition des réactifs.

Dans un ballon à fond rond et à 4 tubulures, équipé d'un agitateur, d'un thermomètre et d'une ampoule à brome on introduit 465 g d'eau, 1,6 g d'acide éthylènediaminetétraacétique, 207,0 g (2,19 moles) d'acide monochloroacétique (99 + %) et 366,2 g de glace. Dans le ballon, on ajoute lentement, tout en refroidissant, 218,4 g (2,73 moles) de solution à 50 % d'hydroxyde de sodium. La température est maintenue entre 35 et 40°C pendant l'addition de l'hydroxyde de sodium.

On ajoute le plus rapidement possible dans le réacteur 504,0 g (1,81 mole) de coco imidazoline ayant été préalablement fondue à une température comprise entre 65 et 70°C. La température est maintenue en dessous de 50°C pendant toute l'addition qui est terminée au bout de 20 minutes. Le mélange réactionnel passe par une phase de gel avant de devenir un liquide limpide. La température de la réaction est maintenue à 50°C pendant 2 heures une fois que l'addition de la coco imidazoline est terminée. La température est alors portée à 75°C et l'on ajoute l'hydroxyde de sodium en trois fois.

On introduit 33,6 g (0,42 mole) de solution d'hydroxyde de sodium à 50 % et on maintient le mélange réactionnel pendant 15 minutes à 75°C. On ajoute encore 33,6 g (0,42 mole) et on maintient la température pendant 15 minutes à 75°C et, ensuite, on introduit 25,08 g (0,31 mole) d'hydroxyde de sodium à 50 %. A l'issue de ces additions, le pH est de 9,3. Le mélange réactionnel est maintenu à 75°C pendant 3 heures supplémentaires. Pendant tout ce temps, on détermine le pH de la réaction toutes les 30 minutes. Si le pH est inférieur à 8,5, on ajoute de l'hydroxyde de sodium pour l'amener à 9,5. Au bout de 3 heures, on détermine la teneur en chlorure, c'est-à-dire en chlorure minéral divisée par la quantité de chlorure présente dans les réactifs. On détermine le rapport en mesurant la teneur en chlorure d'un échantillon et en faisant bouillir ensuite l'échantillon dans de l'hydroxyde de sodium de façon à libérer tout le chlore ayant une liaison organique ; on détermine ainsi la totalité du chlore présent dans l'échantillon. Si le rapport du chlore est de 0,99, on considère que la réaction est complète ; s'il est inférieur à 0,99, on considère qu'une alcoylation quasiment complète n'a pas eu lieu et on continue la réaction à 75°C. Lorsque le rapport du chlorure est égal ou supérieur à 0,99, on considère que la réaction est complète et on porte alors le mélange à une température comprise entre 90 et 95°C pendant 2 heures. Après avoir vérifié de nouveau le rapport du chlore, on refroidit à 60°C et on introduit 146,0 g d'eau afin d'obtenir une teneur en matières solides comprise entre 44 et 45 %.

Le produit analysé contient tel quel 43,9 % de matières solides, 0,76 % d'acide glycolique, moins de 50 ppm de monochloroacétate de sodium et 6,8 % de chlorure de sodium, ou 2,1 % d'acide glycolique, 18,7 % de chlorure de sodium et moins de 115 ppm de monochloroacétate par rapport à la quantité totale en poids de matières actives. L'analyse par résonance magnétique nucléaire au ¹³C indique que le produit est un composé de Formule II, "R" étant un radical coco. Il ne contient pas d'amido amines non alcoylées. Cela est confirmé par la chromatographie capillaire en phase gazeuse effectuée par rajout d'un produit pur non alcoylé obtenu par séparation chromatographique. Le produit présente des caractéristiques tensio-actives de tout premier plan et l'on s'attend à ce que ses propriétés irritantes soient très faibles.

Soumis au test de la hauteur de mousse selon Ross Miles comme indiqué dans ASTM D1173 et comparé à un produit du commerce, le Miranol CM de Rhône-Poulenc Inc., le produit est considéré comme un agent moussant. On évalue la mousse et on obtient les résultats donnés au tableau I suivant :

**TABLEAU I**

| Hauteur de mousse selon Ross Miles à 25°C - 0, 1 % de matière active - au temps zéro - | | | | |
|---|---|---|---|---|
| ECHANTILLON/pH | pH 9,8 | | pH 7,1 | |
| MIRANOL CM | 14,3 cm | | 3,5 cm | |
| EXEMPLE 1 | 14,7 cm | | 15,3 cm | |
| | | | | |

| Hauteur de mousse selon Ross Miles à 25°C - 0,1% de matière active - - 300ppm d'eau dure - pH 7 - | | | | |
|---|---|---|---|---|
| ECHANTILLON/DUREE (mn) | | au temps zéro | | après 5 minutes |
| MIRANOL CM | | 2,7 cm | | 2,45 cm |
| EXEMPLE 1 | | 15,7 cm | | 15,6 cm |

Il résulte clairement des résultats ci-dessus que le produit de l'invention présente, que ce soit dans de l'eau dure ou dans de l'eau douce, une activité de surface supérieure. Le produit du commerce présente, au contraire, notamment dans l'eau dure, une diminution de son activité de surface. Cela signifie que le produit du commerce est contaminé par une matière non alcoylée.

### EXEMPLE 2

On procède comme dans l'exemple 1 en mettant en oeuvre différents rapports molaires entre le monochloroacétate de sodium (SMCA) et l'imidazoline. Les résultats qui figurent dans le Tableau II ci-dessous font apparaître une diminution de la formation d'acide glycolique et l'absence d'amido amine non alcoylée.

**TABLEAU II**

| essai | SMCA/imidazoline molaire | amidoamine alkylée (amidoamine non alkylée) | matière active (% en poids) |
|---|---|---|---|
| 1 | 1,10 | >99,9 (<0,1) | 38,3 |
| 2 | 1,15 | >99,9 (<0,1) | 37,2 |
| 3 | 1,20 | >99,9 (<0,1) | 35,2 |
| 4 | 1,40 | >99,9 (<0,1) | 36,2 |
| | | | |

| essai | acide glycolique % en poids / milieu tel quel (/ à la matière active) | NaCl % en poids / milieu tel quel (/ à la matière active) | imidoamine % en poids / à la matière active |
|---|---|---|---|
| 1 | 0,70 (1,83) | 6,45 (16,8) | <0,25 |
| 2 | 0,76 (2,04) | 6,5 (17,4) | <0,25 |
| 3 | 0,94 (2,67) | 6,8 (19,3) | <0,28 |
| 4 | 1,61 (4,45) | 7,1 (19,6) | <0,28 |

### EXEMPLE 3

On procède comme dans l'exemple 1 en mettant en oeuvre une addition contrôlée de NaOH afin d'obtenir un pH constant durant toute la réaction. Après avoir ajouté la coco imidazoline au monochloroacétate de sodium et chauffé pendant 2 heures à 75°C, on introduit une solution aqueuse à 50 % d'hydroxyde de sodium (92,2 g, 1,15 mole) à une vitesse suffisante pour maintenir le pH de la réaction entre 9,5 et 10. Le pH est contrôlé par un pH-mètre. La température réactionnelle est maintenue à 75°C. L'addition terminée, la réaction est maintenue à 75°C pendant encore une heure. L'analyse par chromatographie en phase gazeuse montre que le produit a une teneur en amido amine non alcoylée inférieure à 0,2 %.

### EXEMPLE 4

Cet exemple est réalisé selon la présente invention en mettant en oeuvre une addition simultanée d'acide chloroacétique et d'hydroxyde de sodium. On émet l'hypothèse selon laquelle, dans ce procédé, l'imidazoline est transformée dans un premier temps en amine secondaire qui, dans un second temps, est alcoylée par la lente addition concomitante d'agent d'alcoylation et d'hydroxyde de sodium.

Dans un ballon à 5 tubulures, équipé d'un agitateur, d'un thermomètre, de deux ampoules et d'un pH-mètre, on introduit 75 g d'eau et 70 g (0,25 mole) de coco imidazoline. On porte le mélange réactionnel à une température de 80°C. On ajoute simultanément à la solution, tout en maintenant le pH entre 9 et 10, 56,7 g (0,3 mole) d'acide chloroacétique aqueux à 50 % et 40 g (0,5 mole) de solution d'hydroxyde de sodium à 50 %. L'addition est effectuée en 1 heure. Le mélange est agité pendant deux heures supplémentaires à 80°C. Le rapport du chlore est de 0,99. L'analyse par RMN indique une absence d'amido amine non alcoylée, ce qui est confirmé ultérieurement par la chromatographie en phase gazeuse.

### EXEMPLE 5

Cet exemple constitue l'illustration du procédé dans lequel l'imidazoline est transformée, dans un premier temps, en amine secondaire et, dans un second temps, alcoylée par la lente addition concomitante de l'agent d'alcoylation et d'hydroxyde de sodium.

Dans un réacteur de 2 litres, équipé de deux pompes pour réactifs, d'un agitateur tournant à environ 450 t / mn, muni de pales en polytetrafluoroéthylène et d'une tige, d'un système de contrôle de la réaction utilisant un thermocouple en verre et un système de contrôle de la température, et d'un système d'aspersion d'azote à faible balayage, on introduit 380 g (1,38 mole) de coco imidazoline, 380 g d'eau et 2 g d'une solution d'hydroxyde de sodium à 47 %. On chauffe le mélange à 85°C en agitant et en procédant à une aspersion d'azote. Après avoir atteint 85°C, on porte la température à 95°C en 1 heure. Le pH est de 9,2. Dès que l'on a atteint 95°C, on commence à introduire lentement 675 g d'une solution de monochloroacétate de sodium à 25 % (1,45 mole - rapport molaire de 1,05 : 1). L'addition se déroule à raison de 2,8 g/minute (moyenne de dix réactions) ce qui équivaut à 5 ml/minute. Au total, l'addition dure 244 minutes. Environ 1 heure après avoir démarré l'addition du monochloroacétate de sodium, on ajoute 264 g (1,65 mole - rapport molaire 1,19) d'une solution d'hydroxyde de sodium à 25 % à raison de 0,9 g/minute ce qui équivaut à 5,6 ml/minute. La durée totale de l'addition est de 293 minutes. Pendant cette addition, le pH varie entre 9,2 et 9,7. Lorsque l'addition est terminée, on chauffe le mélange réactionnel à une température comprise entre 97 et 100°C pendant 2 heures et on laisse refroidir. Le pH est neutralisé à l'aide d'acide chlorhydrique. Le rendement en produit de la réaction est de 1 683 g. Le produit analysé par chromatographie en phase gazeuse et HPLC contient 36,5 % de matières solides, moins de 0,9 % de matière non alcoylée, 0,8 % d'acide glycolique et 7,2 % de chlorure de sodium par rapport au poids de produit tel quel. Cela correspond à 26,1 % de chlorure de sodium, 2,9 % d'acide glycolique et 3,3 % de matière non alcoylée par rapport aux matières actives.

### EXEMPLE 6

Dans un réacteur revêtu de verre, équipé d'un agitateur, d'un condenseur, d'une arrivée et d'une évacuation rapide d'azote, d'un chauffage à la vapeur, d'un refroidissement par eau et de deux pompes doseuses, on introduit 225 parties de coco imidazoline (poids moléculaire moyen 268), 200 parties d'eau et 2 parties d'une solution d'hydroxyde de sodium à 25 %. On chauffe le mélange à la vapeur jusqu'à ce que l'on atteigne 85°C et, ensuite, pendant 1 heure, on applique la vapeur de façon intermittente jusqu'à ce que l'on atteigne lentement une température de 95°C. Après avoir atteint cette température de 95°C, on commence à introduire lentement 445 parties d'une solution aqueuse de monochloracétate de sodium à 25 % (rapport molaire SMCA/imidazoline de 1,2/1). La vitesse moyenne de l'addition est de 95 parties/heure. Une heure après avoir démarré l'introduction du SMCA, on commence à ajouter 150 parties d'une solution aqueuse d'hydroxyde de sodium à 25 % (1,19 équivalents molaires). La solution d'hydroxyde de sodium est introduite par incréments à une vitesse approximative de 16 parties par heure à l'aide d'un système d'introduction modulée permettant de maintenir le pH entre 8,5 et 9,5.

La température est maintenue à 95°C pendant toute la réaction, sauf à la fin de la réaction où la température est portée à 98°C pour transformer le SMCA résiduel en acide glycolique.

Après neutralisation, l'analyse donne les résultats suivants :
- Matières solides 35,9 %
- pH (20 %) 8,42
- Color
• Gardner 2
• Lovibond 11 Y - 2,2 R

| | % / au poids de produit tel quel | % / au poids de matières actives |
|---|---|---|
| NaCl | 7,19 | 26,5 |
| Amido amine | 0,86 | 3,2 |
| SMCA | < 5 ppm | |
| Acide glycolique | 0,8 | 2,95 |
| Azote | 2,2 | |

### EXEMPLE 7

Cet exemple est un exemple comparatif qui montre l'effet exercé sur la présence d'espèces non alcoylées lorsque l'on ne respecte pas les conditions de l'invention. A 116,6 g (1,0 mole) de chloroacétate de sodium en solution dans 350 g d'eau, on ajoute 92,8 g (1,16 mole) d'une solution d'hydroxyde de sodium à 50 %. On ajuste la température de la réaction à 35°C et on ajoute rapidement 278 g (1,0 mole) de coco imidazoline. La température de la réaction varie entre 85 et 90°C et est maintenue à cette température pendant 3 heures. On ajuste le liquide limpide à 45 % de matières solides et on fait une analyse. L'analyse par chromatographie en phase gazeuse montre que le produit contient de l'amido amine non alcoylée. Le rapport du produit alcoylé au produit non alcoylé est de 75/25.

### EXEMPLE 8

Cet exemple est un exemple comparatif qui met en oeuvre l'exemple 1 du brevet US n° 4 269 730. Ce brevet enseigne que la réaction entre l'imidazoline et le sel de chloroacétate est conduite à un pH inférieur à 9 (pH 7,5 - 8,5) afin d'obtenir une bétaïne cyclique stable à des pH inférieurs à 9. Les réactions enseignées dans ce brevet sont effectuées en vue de préparer la bétaïne en maintenant un pH qui n'est que légèrement alcalin. Le procédé selon l'Exemple 1 du brevet US n° 4 269 730 est mis en oeuvre conformément aux conditions énoncées. L'analyse par chromatographie en phase gazeuse montre que le produit obtenu présente un rapport entre l'amido amine alcoylée et l'amido amine non alcoylée de l'ordre de 85/15. Une seconde expérience mettant en oeuvre un rapport molaire de 1,2 : 1 entre le monoachloroacétate de sodium et l'imidazoline donne un produit présentant un rapport du produit alcoylé au produit non alcoylé (analyse par chromatographie en phase gazeuse) de 70/30.

## Revendications

1. Composition tensio-active à base d'amphoacétate dérivé de l'imidazoline substituée et contenant comme composant principal un composé de formule
R - C(O) - N(H) - CH₂CH₂ - N - [(CH₂)_{N}X] - CH₂COOM
dans laquelle:
- R représente des radicaux aliphatiques contenant environ de 5 à 19 atomes de carbone,
- N est un nombre entier de 2 à 4 ,
- X représente OH ou NH₂, et
- M est un métal,
ladite composition contenant:
- moins de 3,5 % d'amide non alcoylée,
- moins de 4,5 % d'acide glycolique, et
- moins de 27 % d'un sel d'halogénure de métal alcalin, en poids par rapport aux matières actives.

2. Composition selon la revendication 1 dans laquelle ladite composition contient moins de 2,0 % d'amide non alcoylée, en poids par rapport aux matières actives.

3. Composition selon la revendication 1 dans laquelle ladite composition contient moins 3,5 % d'acide glycolique, en poids par rapport aux matières actives.

4. Composition selon la revendication 2 dans laquelle ladite composition contient moins 3,5 % d'acide glycolique, en poids par rapport aux matières actives.

5. Composition selon la revendication 1 dans laquelle ladite composition contient, en outre, moins de 23 % d'un sel d'halogénure de métal alcalin, en poids par rapport aux matières actives.

6. Composition selon la revendication 4 dans laquelle ladite composition contient, en outre, moins de 23 % d'un sel d'halogénure de métal alcalin, en poids par rapport aux matières actives.

7. Composition selon la revendication 1 dans laquelle X représente OH.

8. Composition selon la revendication 1 dans laquelle R dérive d'un acide choisi dans le groupe comprenant les acides gras de l'huile de coco, les acides gras de l'huile de palme, les acides caprique, caproïque, caprylique, hexadécadiénoïque, laurique, linoléique, linolénique, margarique, myristique, myristoléique, oléique, palmitique, palmitoléique et stéarique et leurs mélanges.

9. Composition selon la revendication 1 dans laquelle R dérive des acides gras de l'huile de coco.

10. Composition selon la revendication 1 dans laquelle M est un métal alcalin.

11. Composition selon la revendication 1 dans laquelle R dérive des acides gras de l'huile de coco, N est 2, X représente OH et M est le sodium.

12. Composition selon l'une des revendications précédentes, **caractérisée en ce qu'**elle comprend au moins 35,2 % en poids de matières actives.

13. Composition selon l'une des revendications précédentes, **caractérisée en ce qu'**elle comprend moins de 7,2% en poids en sels de métaux alcalins.

14. Composition selon l'une des revendications précédentes, **caractérisée en ce que** qu'elle comprend moins de 20% en poids en sels de métaux alcalins, par rapport à la matière active.

15. Composition selon l'une des revendications précédentes, **caractérisée en ce qu'**elle comprend moins de 115 ppm d'un acide monohalogénoacétique résiduel.

16. Utilisation de la composition selon l'une des revendications précédentes, dans des produits cosmétiques.

17. Utitisation selon la revendication précédente, dans des shampoings, les shampoings corporels, les gels et lotions de mise en forme de la chevelure, les crèmes et lotions pour la peau, les crèmes et lotions de démaquillage.

18. Utilisation selon l'une des revendications précédentes, en tant que tensioactif compatible avec des tensioactifs anioniques.

## Claims

1. An amphoacetate-based surfactant composition derived from substituted imidazoline and containing, as the main component, a compound of formula:
R - C(O) - N (H) - CH₂CH₂ - N - [ (CH₂) _{N}X] - CH₂COOM
in which:
- R represents aliphatic radicals containing about 5 to 19 carbon atoms;
- N is an integer from 2 to 4;
- X represents OH or NH₂; and
- M is a metal,
said composition containing:
- less than 3.5% of unalkylated amide;
- less than 4.5% of glycolic acid; and
- less than 27% of an alkaline metal halide salt,
by weight relative to the active ingredients.

2. Composition according to Claim 1, in which said composition contains less than 2.0% of unalkylated amide, by weight relative to the active ingredients.

3. Composition according to Claim 1, in which said composition contains less than 3.5% of glycolic acid, by weight relative to the active ingredients.

4. Composition according to Claim 2, in which said composition contains less than 3.5% of glycolic acid, by weight relative to the acid ingredients.

5. Composition according to Claim 1, in which said composition contains, in addition, less than 23% of an alkali metal halide salt, by weight relative to the active ingredients.

6. Composition according to Claim 4, in which said composition contains, in addition, less than 23% of an alkali metal halide salt, by weight relative to the active ingredients.

7. Composition according to Claim 1, in which X represents OH.

8. Composition according to Claim 1, in which R derives from an acid chosen from the group comprising coconut oil fatty acids, palm oil fatty acids, capric, caproic, caprylic, hexadecadienoic, lauric, linoleic, linolenic, margaric, myristic, myristoleic, oleic, palmitic, palmitoleic and stearic acids and mixtures thereof.

9. Composition according to Claim 1, in which R derives from coconut oil fatty acids.

10. Composition according to Claim 1, in which M is an alkali metal.

11. Composition according to Claim 1, in which R derives from coconut oil fatty acids, N is 2, X represents OH and M is sodium.

12. Composition according to one of the preceding claims, **characterized in that** it comprises at least 35.2% by weight of active ingredients.

13. Composition according to one of the preceding claims, **characterized in that** it comprises less than 7.2% by weight of alkali metal salts.

14. Composition according to one of the preceding claims, **characterized in that** it comprises less than 20% by weight of alkali metal salts, relative to the active ingredients.

15. Composition according to one of the preceding claims, **characterized in that** it comprises less than 115 ppm of a residual monohaloacetic acid.

16. Use of the composition according to one of the preceding claims, in cosmetic products.

17. Use according to the preceding claim, in shampoos, body shampoos, hair shaping gels and lotions, skin creams and lotions and makeup removal creams and lotions.

18. Use according to one of the preceding claims, as a surfactant that is compatible with anionic surfactants.

## Patentansprüche

1. Grenzflächenaktive Zusammensetzung die auf von substituiertem Imidazolin abgeleitetem Amphoacetat basiert und als Hauptkomponente eine Verbindung der Formel
R - C (O) - N (H) - CH₂CH-₂ - N - [(CH₂) _{N}X] - CH₂COOM
worin:
- R für aliphatische Reste mit ungefähr 5 bis 19 Kohlenstoffatomen steht,
- N für eine ganze Zahl von 2 bis 4 steht,
- X für OH oder NH₂ steht und
- M für ein Metall steht,
wobei die Zusammensetzung
- weniger als 3,5 Gew.-% nichtalkyliertes Amid,
- weniger als 4,5 Gew.-% Glykolsäure und
- weniger als 27 Gew.-% eines Alkalimetallhalogenidsalzes,
bezogen auf die Wirkstoffe,
enthält.

2. Zusammensetzung nach Anspruch 1, die weniger als 2,0 Gew.-% nichtalkyliertes Amid, bezogen auf die Wirkstoffe, enthält.

3. Zusammensetzung nach Anspruch 1, die weniger als 3,5 Gew.-% Glykolsäure, bezogen auf die Wirkstoffe, enthält.

4. Zusammensetzung nach Anspruch 2, die weniger als 3,5 Gew.-% Glykolsäure, bezogen auf die Wirkstoffe, enthält.

5. Zusammensetzung nach Anspruch 1, die außerdem weniger als 23 Gew.-% eines Alkalimetallhalogenidsalzes, bezogen auf die Wirkstoffe, enthält.

6. Zusammensetzung nach Anspruch 4, die außerdem weniger als 23 Gew.-% eines Alkalimetallhalogenidsalzes, bezogen auf die Wirkstoffe, enthält.

7. Zusammensetzung nach Anspruch 1, worin X für OH steht.

8. Zusammensetzung nach Anspruch 1, worin R sich von einer Säure aus der Gruppe umfassend Kokosölfettsäuren, Palmölfettsäuren, Caprinsäure, Capronsäure, Caprylsäure, Hexadecadiensäure, Laurinsäure, Linolsäure, Linolensäure, Margarinsäure, Myristinsäure, Myristoleinsäure, Ölsäure, Palmitinsäure, Palmitoleinsäure und Stearinsäure und Mischungen davon ableitet.

9. Zusammensetzung nach Anspruch 1, worin R sich von Kokosölfettsäuren ableitet.

10. Zusammensetzung nach Anspruch 1, worin M für ein Alkalimetall steht.

11. Zusammensetzung nach Anspruch 1, worin R sich von Kokosölfettsäuren ableitet, N für 2 steht, X für OH steht und M für Natrium steht.

12. Zusammensetzung nach einem der vorhergehenden Ansprüche, **dadurch gekennzeichnet, daß** sie mindestens 35,2 Gew.-% Wirkstoffe enthält.

13. Zusammensetzung nach einem der vorhergehenden Ansprüche, **dadurch gekennzeichnet, daß** sie weniger als 7,2 Gew.-% Alkalimetallsalze enthält.

14. Zusammensetzung nach einem der vorhergehenden Ansprüche, **dadurch gekennzeichnet, daß** sie weniger als 20 Ges.-% Alkalimetallsalze, bezogen auf die Wirkstoffe, enthält.

15. Zusammensetzung nach einem der vorhergehenden Ansprüche, **dadurch gekennzeichnet, daß** sie weniger als 115 ppm restliche Monohalogenessigsäure enthält.

16. Verwendung der Zusammensetzung nach einem der vorhergehenden Ansprüche in Kosmetikprodukten.

17. Verwendung nach dem vorhergehenden Anspruch in Shampoos, Körpershampoos, Haarstyling-Gelen und -Lotionen, Hautcremes und -lotionen und Abschminkcremes und -lotionen.

18. Verwendung nach einem der vorhergehenden Ansprüche als mit anionischen Tensiden verträgliches Tensid.
